# EUROPEAN PATENT APPLICATION

(11) **EP 3 533 470 A1**
(43) Date of publication of application: **04.09.2019**
(21) Application number: 17864054.6
(22) Date of filing: 31.10.2017
(51) Int. Cl.: A61K 45/00, A61K 31/472, A61P 35/00

(54) **PHARMACEUTICAL COMPOSITION FOR INDUCING PROGRAMMED CELL DEATH IN CANCER CELLS**

(30) Priority: 31.10.2016 JP 2016213755
(71) Applicant: Kyoto University, Kyoto-shi, Kyoto 606-8501 (JP)
(72) Inventor: HAGIWARA Masatoshi, Kyoto-shi, Kyoto 606-8501 (JP); TOYOMOTO Masayasu, Kyoto-shi, Kyoto 606-8501 (JP); HOSOYA Takamitsu, Tokyo 113-8510 (JP); YOSHIDA Suguru, Tokyo 113-8510 (JP)
(74) Representative: Dehns
(86) International application number: PCT/JP2017/039426
(87) International publication number: WO 2018/079855

(57) **Abstract**

A method for inducing programmed cell death in cancer cells and a pharmaceutical composition therefor are provided.

Provided is a method for inducing programmed cell death in cancer cells, the method including enhancing production of nitric oxide in cancer cells. Provided is a pharmaceutical composition for inducing programmed cell death in cancer cells, the pharmaceutical composition containing, as an active component, a compound that enhances production of nitric oxide in cancer cells. Provided is use of a compound that enhances production of nitric oxide in cancer cells, in a method for preventing, improving, suppressing progression of, and/or treating cancers.

## Description

### Technical Field

The present disclosure relates to a pharmaceutical composition for inducing programmed cell death in cancer cells, use thereof, and a method for inducing programmed cell death in cancer cells.

### Background Art

Patent Document 1 discloses a therapeutic agent for an arterial occlusive disease containing an endothelial nitric oxide production accelerator and/or an endothelial nitric oxide synthase activator.

Patent Document 2 discloses an antitumor drug containing a component that enhances production of nitric oxide by macrophages.

### Prior Art Documents

### Patent Document

[Patent Document 1] JP 2007-186434A
[Patent Document 2] JP 2012-153612A

### Disclosure of Invention

### Problem to be Solved by the Invention

A method for inducing programmed cell death in cancer cells and a pharmaceutical composition therefor are provided.

### Means for Solving Problem

In one aspect, the present disclosure relates to a method for inducing programmed cell death in cancer cells, the method including enhancing production of nitric oxide in cancer cells.

In another aspect, the present disclosure relates to a pharmaceutical composition for inducing programmed cell death in cancer cells, the pharmaceutical composition containing, as an active component, a compound that enhances production of nitric oxide in cancer cells.

In another aspect, the present disclosure relates to use of a compound that enhances production of nitric oxide in cancer cells, in a method for preventing, improving, suppressing progression of, and/or treating cancers.

In another aspect, the present disclosure relates to a pharmaceutical composition for preventing, improving, suppressing progression of, and/or treating diseases attributable to a decrease in the production of nitric oxide in cells, and to a method therefore.

In another aspect, the present disclosure relates to a pharmaceutical composition for preventing, improving, suppressing progression of, and/or treating a lifestyle disease and/or metabolic syndrome, and to a method therefore.

### Effects of the Invention

According to the present disclosure, in one aspect, it is possible to induce programmed cell death in cancer cells.

### Brief Description of Drawings

FIG. 1 is a graph showing the results of the enhancement of NO production in cancer cells by a compound according to the present disclosure.
FIG. 2 is a photograph of the results of living cell staining with CFSE-DA showing an antitumor effect of a compound according to the present disclosure.
FIG. 3 is a graph of the results of the number of living cells with WST-8 assay showing the antitumor effect of a compound according to the present disclosure.
FIG. 4 is a diagram of the results of flow cytometry dot plots showing the activation of caspase 3 by compounds according to the present disclosure.
FIG. 5 is a diagram of the results of flow cytometry dot plots showing NO dependency of the activation of caspase 3 by a compound according to the present disclosure.
FIG. 6 is a graph of the results of WST-8 assays indicating a relationship between the antitumor effect due to a compound according to the present disclosure and a glucose starvation.

### Description of the Invention

In one aspect, the present disclosure is based on new findings that facilitating the production of nitric oxide (NO) in cancer cells makes it possible to induce programmed cell death in the cancer cells.

Although a detailed mechanism by which, as a result of production of NO being enhanced in cancer cells, programmed cell death is induced in the cancer cells is not clear, a mechanism is thought to be as follows.

If the production of NO increases in cells, reduced glutathione and thioredoxin are used to maintain a redox state in cells, and NADPH is consumed to re-reduce reduced glutathione and thioredoxin. Then, in order to replenish a reduced amount of NADPH, cells consume glucose to produce NADPH (the pentose phosphate pathway). Also, an increase in NO in cells induces a decrease in the number of molecules that inhibit thioredoxin activity. As a result, an antioxidant effect is enhanced in normal cells.

On the other hand, in cancer cells, ATP is normally produced through glycolysis, instead of oxidative phosphorylation. Thus, cancer cells consume a large amount of glucose (the Warburg effect). It is inferred that, if the production of NO is enhanced in such cancer cells, the cancer cells further consume glucose in order to replenish a reduced amount of NADPH, and as a result, the cancer cells are deprived of glucose and apoptosis is induced in the cancer cells. However, the present disclosure is not limited to these mechanisms.

### Method for inducing programmed cell death in cancer cells

Thus, in one aspect, the present disclosure relates to a method for inducing programmed cell death in cancer cells, the method including enhancing production of nitric oxide (NO) in cancer cells (also referred to as an "induction method according to the present disclosure").

"Cancer cells" refer to "human cells and cells of an animal other than a human" in the present disclosure. Also, "cancer cells" refer to "malignant tumor cells" in the present disclosure.

In the present disclosure, "programmed cell death" includes apoptosis, autophagic cell death, and necrotic programmed cell death. In one or more embodiments, apoptosis can be confirmed through detection of the activation of a maker of apoptosis (for example, caspase 3). In one or more embodiments, autophagic cell death can be confirmed due to the enhancement of expression of a marker of autophagy (for example, LC3-II).

In one or more embodiments that are not limited, an example of a method for enhancing the production of NO in cancer cells is causing a compound capable of enhancing the production of NO in cancer cells to come into contact with cancer cells. In one or more embodiments that are not limited, enhancement of production of NO in cancer cells is enhancement of production of NO via a transmembrane protein. Examples of the transmembrane protein include G protein-coupled receptors (GPCRs) and other channels, and in one or more embodiments, examples thereof include S1P3, ADOR1, TRPV1, TRPV3, and TRPA1, but are not limited thereto. In one or more embodiments, examples of the compound capable of enhancing the production of NO in cancer cells include compounds that can activate nitric oxide synthases (NOSs) and the like in cancer cells and induce the production of NO. In one or more embodiments, examples of this compound include compounds according to the present disclosure, which will be described later.

In one or more embodiments, an example of a method for enhancing the production of NO in cancer cells is a method using a compound according to the present disclosure or a pharmaceutical composition according to the present disclosure, which will be described later. That is, the production of NO in cancer cells can be enhanced by causing a compound according to the present disclosure or a pharmaceutical composition according to the present disclosure to come into contact with cancer cells, or by administering, to a living body having cancer cells, a compound according to the present disclosure or a pharmaceutical composition according to the present disclosure. A method according to this aspect can be used in vivo, in vitro, and ex vivo.

In one or more embodiments that are not limited, the production of NO in cancer cells can be detected using an NO indicator. Specifically, although a method described in examples may be used, the present disclosure is not limited thereto.

In one or more embodiments, examples of cancer cells in which programmed cell death can be induced by enhancing the production of NO in cancer cells include cancer cells that are highly dependent on glucose. In one or more embodiments, examples of cancer cells that are highly dependent on glucose include cancer cells whose ATP production is dependent on glycolysis rather than oxidative phosphorylation, and cancer cells in which the Warburg effect is expressed.

Cancer cells that are highly dependent on glucose can be confirmed through PET examination using a PET (positron emission tomography) probe (¹⁸F-FDG; 2-fluoro-2-deoxy-D-glucose), for example.

Thus, in one or more embodiments, examples of cancer cells in which programmed cell death can be induced by enhancing the production of NO in cancer cells include PET-positive cancer cells.

There is no particular limitation on the type of cancer cells or the focus of cancers in which programmed cell death can be induced by enhancing the production of NO in cancer cells. In one or more embodiments that are not limited, examples thereof include brain tumors, glioblastomas, pancreatic ductal carcinomas, rhabdomyosarcomas, lung cancer, colon cancer, skin cancer, prostate cancer, breast cancer, and ovarian cancer.

In one or more embodiments of an induction method according to the present disclosure, from the viewpoint of increasing the efficiency of induction of programmed cell death, it is preferable to introduce, into cancer cells, a glucose derivative that cannot be metabolized through glycolysis, or a glucose derivative that serves as an intermediate metabolite that cannot be metabolized through glycolysis, in addition to enhancement of production of nitric oxide in cancer cells. An example of such a glucose derivative is 2-deoxy-D-glucose (2-DG). Cells take up a large amount of 2-DG, and 2-DG-6-phosphoric acid that cannot be metabolized through glycolysis is accumulated in the cells in the metabolic process. As a result, glycolysis and the pentose phosphate pathway are inhibited, and the production of NADPH decreases, and the efficiency of induction of programmed cell death by NO is increased.

In one or more embodiments of an induction method according to the present disclosure, from the viewpoint of increasing the efficiency of induction of programmed cell death, it is preferable to reduce the production of NADPH in cancer cells, in addition to enhancement of the production of nitric oxide in cancer cells.

The production of NADPH can be reduced by inhibiting glycolysis, the pentose phosphate pathway, a malate dehydrogenase, an isocitrate dehydrogenase, an amino acid transporter, and the like.

In one or more embodiments, a substance that exhibits hexose inhibitory activity can be used as an inhibitor of the pentose phosphate pathway.

In one or more embodiments, examples of a pharmaceutical agent capable of inhibiting glycolysis include lonidamine, 3-bromopyruvic acid, and imatinib.

In one or more embodiments of an induction method according to the present disclosure, it is preferable that the method includes causing a substrate of a nitric oxide synthase and/or a substance that serves as a substrate of a nitric oxide synthase to come into contact with cancer cells, or administering, to a living body having cancer cells, a substrate of a nitric oxide synthase and/or a substance that serves as a substrate of a nitric oxide synthase, in addition to enhancement of production of nitric oxide in cancer cells using a compound according to the present disclosure or a pharmaceutical composition according to the present disclosure.

In one or more embodiments, examples of a substrate of a nitric oxide synthase and/or a substance that serves as a substrate of a nitric oxide synthase include substances that are consumed by this enzyme, and examples thereof include arginine, citrulline, and argininosuccinic acid.

Also, the present disclosure relates to the following one or more embodiments that are not limited.
[A1] A method for inducing programmed cell death in cancer cells, the method including enhancing production of nitric oxide in cancer cells.
[A2] The method according to [A1], in which enhancement of the production of nitric oxide is enhancement of production of nitric oxide via a transmembrane protein.
[A3] The method according to [A1] or [A2], in which the cancer cells are cancer cells whose ATP production is dependent on glycolysis rather than oxidative phosphorylation.
[A4] The method according to any of [A1] to [A3], in which the cancer cells are cancer cells in which the Warburg effect is expressed.
[A5] The method according to any of [A1] to [A4], in which production of nitric oxide is enhanced by causing a compound according to the present disclosure or a pharmaceutical composition according to the present disclosure to come into contact with cancer cells, or administering, to a living body having cancer cells, a compound according to the present disclosure or a pharmaceutical composition according to the present disclosure.
[A6] The method according to any of [A1] to [A5], further including introducing, into cancer cells, an agent that reduces production of NADPH in cancer cells.
[A7] The method according to any of [A1] to [A5], further including introducing, into cancer cells, an agent that performs at least one inhibition selected from the group consisting of inhibition of glycolysis, inhibition of a pentose phosphate pathway, inhibition of a malate dehydrogenase, inhibition of an isocitrate dehydrogenase, and inhibition of an amino acid transporter.
[A8] The method according to any of [A1] to [A5], further including introducing, into cancer cells, an inhibitor of glycolysis.
[A9] The method according to any of [A1] to [A5], further including introducing, into cancer cells, a substance having hexokinase inhibitory activity.
[A10] The method according to any of [A1] to [A5], further including introducing, into cancer cells, a glucose derivative that cannot be metabolized through glycolysis or a glucose derivative that serves as an intermediate metabolite that cannot be metabolized through glycolysis.
[A11] The method according to any of [A1] to [A10], in which programmed cell death is apoptosis.
[A12] The method according to [A5] to [A11], further including introducing, into cancer cells, a substrate of a nitric oxide synthase and/or a substance that serves as a substrate of a nitric oxide synthase.
[A13] The method according to [A5] to [A11], in which enhancement of production of NO further includes causing a substrate of a nitric oxide synthase and/or a substance that serves as a substrate of a nitric oxide synthase to come into contact with cancer cells, or administering, to a living body having cancer cells, a substrate of a nitric oxide synthase and/or a substance that serves as a substrate of a nitric oxide synthase.

### Pharmaceutical composition for inducing programmed cell death in cancer cells

In one aspect, the present disclosure relates to a pharmaceutical composition for inducing programmed cell death in cancer cells, the pharmaceutical composition containing, as an active component, a compound that enhances production of nitric oxide (NO) in cancer cells (also referred to as a "pharmaceutical composition according to the present disclosure"). It is preferable that the pharmaceutical composition according to the present disclosure contains an effective amount of a compound that enhances production of NO in cancer cells. In one or more embodiments, the effective amount refers to an amount by which programmed cell death can be induced in cancer cells. A "compound that enhances production of NO in cancer cells" will be described later. Cancer cells in which programmed cell death can be induced using a pharmaceutical composition according to the present disclosure are similar to the above-described cancer cells in which programmed cell death can be induced as a result of enhancing production of NO in cancer cells.

In one or more embodiments that are not limited, enhancement of production of NO using a compound that enhances production of NO in cancer cells is enhancement of production of NO via a transmembrane protein. As described above, examples of the transmembrane protein include G protein-coupled receptors (GPCRs) and other channels.

In one or more embodiments, from the viewpoint of increasing the efficiency of induction of programmed cell death, it is preferable that a pharmaceutical composition according to the present disclosure contains or is used in combination with an agent that reduces production of NADPH in cancer cells, in addition to enhancement of production of nitric oxide in cancer cells.

The production of NADPH can be reduced by inhibiting glycolysis, the pentose phosphate pathway, a malate dehydrogenase, an isocitrate dehydrogenase, an amino acid transporter, and the like.

In one or more embodiments, a substance that exhibits hexose inhibitory activity can be used to inhibit the pentose phosphate pathway.

In one or more embodiments, examples of a pharmaceutical agent capable of inhibiting glycolysis include lonidamine, 3-bromopyruvic acid, and imatinib.

In one or more embodiments, a pharmaceutical composition according to the present disclosure preferably contains a substrate of a nitric oxide synthase and/or a substance that serves as a substrate of a nitric oxide synthase or is used in combination with the substrate of a nitric oxide synthase and/or the substance that serves as a substrate of a nitric oxide synthase, in addition to enhancement of production of NO in cancer cells by a compound according to the present disclosure or a pharmaceutical composition according to the present disclosure.

In one or more embodiments, examples of a substrate of a nitric oxide synthase and/or a substance that serves as a substrate of a nitric oxide synthase include substances that are consumed by this enzyme, and examples thereof include arginine, citrulline, and argininosuccinic acid.

From the viewpoint of increasing the efficiency of induction of programmed cell death, the pharmaceutical composition according to the present disclosure may further contain a glucose derivative that cannot be metabolized through glycolysis or a glucose derivative that serves as an intermediate metabolite that cannot be metabolized through glycolysis. An effective amount may be used as the content thereof. In one or more embodiments, an example of the effective amount is an amount by which glycolysis and/or the pentose phosphate pathway of cancer cells can be inhibited.

In one or more embodiments, a known drug preparation technique may be applied to the pharmaceutical composition according to this aspect to have a dosage form that is suitable for an administration form. An example of the administration form is, although is not limited to, oral administration via dosage forms such as tablets, capsules, granules, powders, pills, troches, syrups, and liquid formulations. Alternatively, an example of the administration form is parenteral administration via dosage forms such as injections, liquid formulations, aerosols, suppositories, plasters and pressure sensitive adhesives, cataplasms, lotions, liniments, ointments, and ophthalmic solutions. Although these pharmaceutical preparations are not limited thereto, they may be manufactured using a known method using additive agents such as excipients, lubricants, binders, disintegrants, stabilizing agents, flavoring agents, and diluents.

Examples of the excipient include, but are not limited to, starches such as starch, potato starch, and corn starch, lactose, crystalline cellulose, and calcium hydrogen phosphate. Examples of the coating agent include, but are not limited to, ethyl cellulose, hydroxypropyl cellulose, hydroxypropyl methylcellulose, shellac, talc, carnauba wax, and paraffin. Examples of the binder include, but are not limited to, polyvinylpyrrolidone, macrogol, and compounds that are similar to the above-described excipients. Examples of the disintegrant include, but are not limited to, compounds that are similar to the above-described excipients, chemically-modified starches and celluloses such as croscarmellose sodium, sodium carboxymethyl starch, and crosslinked polyvinylpyrrolidone. Examples of the stabilizing agent include, but are not limited to, *para*-hydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid. Examples of the flavoring agent include, but are not limited to, sweeteners, acidulants, and flavors that are usually used.

Also, although there is no limitation on a solvent, ethanol, phenol, chlorocresol, purified water, distilled water, and the like can be used as a solvent to manufacture liquid formulations, and a surfactant or an emulsifying agent can also be used as needed. Examples of the surfactant or emulsifying agent include, but are not limited to, polysorbate 80, polyoxyl 40 stearate, and lauromacrogol.

A method for using a pharmaceutical composition according to this aspect may change depending on the symptoms, age, administration method, and the like. Although there is no limitation on the usage method, a pharmaceutical composition can be intermittently or continuously administered orally, percutaneously, submucosally, subcutaneously, intramuscularly, intravascularly, intracerebrally, or intraperitoneally such that the concentration of a compound in the body that is an active component and enhances the production of NO in cancer cells is in a range of 100 nM to 1 mM. As a non-limiting embodiment, in the case of oral administration, the pharmaceutical composition is administered to a subject (an adult human if the subject is a human) according to a symptom with 0.01 mg (preferably 0.1 mg) being the lower limit and 2000 mg (preferably 500 mg, more preferably 100 mg) being the upper limit once or over several times in one day, in terms of a compound expressed as a compound that enhances the production of NO in cancer cells. As a non-limiting embodiment, in the case of intravenous administration, a pharmaceutical composition is administered to a subject (an adult human if the subject is a human) according to a symptom with 0.001 mg (preferably 0.01 mg) being the lower limit and 500 mg (preferably 50 mg) being the upper limit once or over several times in one day.

Also, the present disclosure relates to the following one or more embodiments that are not limited.
[B1] A pharmaceutical composition for inducing programmed cell death in cancer cells, the pharmaceutical composition containing, as an active component, a compound that enhances production of nitric oxide in cancer cells.
[B2] The pharmaceutical composition according to [B1], in which enhancement of the production of nitric oxide is enhancement of production of nitric oxide via a transmembrane protein.
[B3] The pharmaceutical composition according to [B1] or [B2], in which the cancer cells are cancer cells whose ATP production is dependent on glycolysis rather than oxidative phosphorylation.
[B4] The pharmaceutical composition according to any of [B1] to [B3], in which the cancer cells are cancer cells in which the Warburg effect is expressed.
[B5] The pharmaceutical composition according to any of [B1] to [B4], in which programmed cell death is apoptosis.
[B6] The pharmaceutical composition according to any of [B1] to [B5], in which a compound that enhances production of nitric oxide in cancer cells is a compound according to the present disclosure, which will be described later.
[B7] The pharmaceutical composition according to any of [B1] to [B6], which further contains or is used in combination with an agent that reduces production of NADPH in cancer cells.
[B8] The pharmaceutical composition according to any of [B1] to [B6], which further contains or is used in combination with an agent that performs at least one inhibition selected from the group consisting of inhibition of glycolysis, inhibition of a pentose phosphate pathway, inhibition of a malate dehydrogenase, inhibition of an isocitrate dehydrogenase, and inhibition of an amino acid transporter.
[B9] The pharmaceutical composition according to any of [B1] to [B6], which further contains or is used in combination with an inhibitor of glycolysis.
[B10] The pharmaceutical composition according to any of [B1] to [B6], which further contains or is used in combination with a substance having hexokinase inhibitory activity.
[B11] The pharmaceutical composition according to any of [B1] to [B10], which further contains or is used in combination with a glucose derivative that cannot be metabolized through glycolysis or a glucose derivative that serves as an intermediate metabolite that cannot be metabolized through glycolysis.
[B12] The pharmaceutical composition according to any of [B1] to [B6], which further contains or is used in combination with a substrate of a nitric oxide synthase and/or a substance that serves as a substrate of a nitric oxide synthase.

### Compound that enhances production of nitric oxide in cancer cells

In one or more embodiments, a "compound that enhances production of nitric oxide (NO) in cancer cells" in the present disclosure refers to a compound represented by Formula (I) below, a prodrug thereof, or a pharmaceutically acceptable salt thereof (also referred to as "a compound according to the present disclosure").

Thus, in one aspect, the present disclosure relates to a compound represented by Formula (I) below, a prodrug thereof, or a pharmaceutically acceptable salt thereof.

Also, in another aspect, the present disclosure relates to use of a compound represented by Formula (I) below, a prodrug thereof, or a pharmaceutically acceptable salt thereof, and a pharmaceutical composition containing the same. The pharmaceutical composition can be used for applications to induce programmed cell death in cancer cells.

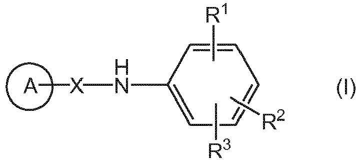

In Formula (I), A represents a substituted or unsubstituted monocyclic, bicyclic, or bicyclic fused aryl group or heteroaryl group,
X represents an oxygen atom, -C(=O)-, -C(=S)-, or -SO₂-,
R¹ represents a hydrogen atom, a substituted or unsubstituted C₁₋₆ alkyl group, a substituted or unsubstituted C₂₋₆ alkenyl group, a substituted or unsubstituted C₂₋₆ alkynyl group, a substituted or unsubstituted C₆₋₁₀ aryl group, a halogen atom, a nitro group, a cyano group, an azido group, a hydroxy group, a substituted or unsubstituted C₁₋₆ alkoxy group, a substituted or unsubstituted C₁₋₆ alkylthio group, a substituted or unsubstituted C₁₋₆ alkyl sulfonyl group, a carboxy group, a formyl group, a substituted or unsubstituted C₁₋₆ alkoxycarbonyl group, an acyl group, an acylamino group, or a sulfamoyl group,
R² represents a hydrogen atom, a substituted or unsubstituted C₁₋₆ alkyl group, or a halogen atom,
R³ represents a hydrogen atom, a substituted or unsubstituted C₁₋₆ alkyl group, a substituted or unsubstituted C₆₋₁₀ aryl group, a halogen atom, a hydroxy group, a substituted or unsubstituted C₁₋₆ alkoxy group, a substituted or unsubstituted C₁₋₆ alkylthio group, a substituted or unsubstituted nitrogen-containing heterocyclic ring, a substituted or unsubstituted fused aromatic heterocyclic ring, or R³ is represented by Formula (II) below,

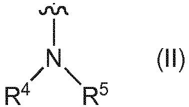

in Formula (II), R⁴ and R⁵ are the same as or different from each other, and each represent a hydrogen atom, a substituted or unsubstituted C₁₋₆ alkyl group, a substituted or unsubstituted nitrogen-containing heterocyclic ring, a substituted or unsubstituted fused aromatic heterocyclic ring, an acyl group, or an acylamino group; or
R⁴ and R⁵ together with the adjacent nitrogen atom each form a substituted or unsubstituted heterocyclic ring, or a substituted or unsubstituted fused aromatic heterocyclic ring; or
R⁴ and R⁵ each represent a substituted or unsubstituted cycloalkylidene amino group, or a substituted or unsubstituted aromatic ring fused cycloalkylidene group.

In Formula (II), an atomic bonding to which a wavy line is attached indicates a portion that binds to Formula (I).

In one or more embodiments, examples of A in Formula (I) above include

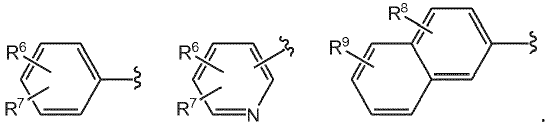

In A represented by the formulae described above, R⁶ to R⁹ are independent from each other, and each represent a hydrogen atom, a substituted or unsubstituted C₁₋₆ alkyl group, a substituted or unsubstituted C₂₋₆ alkenyl group, a substituted or unsubstituted C₂₋₆ alkynyl group, a substituted or unsubstituted C₆₋₁₀ aryl group, a halogen atom, a nitro group, a cyano group, an azido group, a hydroxy group, a substituted or unsubstituted C₁₋₆ alkoxy group, a substituted or unsubstituted C₁₋₆ alkylthio group, a substituted or unsubstituted C₁₋₆ alkyl sulfonyl group, a carboxy group, a formyl group, a substituted or unsubstituted C₁₋₆ alkoxycarbonyl group, an acyl group, an acylamino group, or a sulfamoyl group. Alternatively, R⁶ and R⁷ are adjacent to each other and form a substituted or unsubstituted homocyclic ring or heterocyclic ring.

In one or more embodiments, R⁶ represents a hydrogen atom, a substituted or unsubstituted C₁₋₂ alkyl group, a substituted or unsubstituted C₆₋₁₀ aryl group, a halogen atom, a nitro group, a cyano group, an azido group, a hydroxy group, a substituted or unsubstituted C₁₋₆ alkoxy group, and in one embodiment, R⁷ represents a hydrogen atom, or R⁶ and R⁷ are adjacent to each other and form a substituted or unsubstituted 6-membered homocyclic ring or heterocyclic ring.

In one or more embodiments, R⁸ and R⁹ each represent a hydrogen atom, a substituted or unsubstituted C₁₋₂ alkyl group, a halogen atom, a nitro group, a cyano group, an azido group, a hydroxy group, or a substituted or unsubstituted C₁₋₆ alkoxy group.

In the formulae described above, an atomic bonding to which a wavy line is attached indicates a portion that binds to X in Formula (I).

In one or more embodiments, examples of A in Formula (I) above include

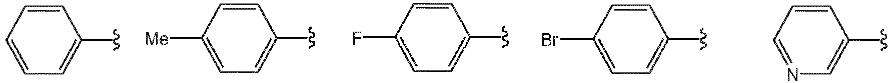

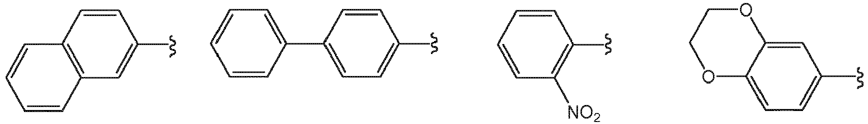

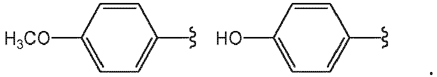

In one or more embodiments, examples of R¹ in Formula (I) above include a hydrogen atom, a C₁₋₃ alkyl group, a halogen-substituted C₁₋₃ alkyl group, a halogen atom, a nitro group, a cyano group, an azido group, a hydroxy group, and a C₁₋₆ alkoxy group. In one or more embodiments, R¹ represents a hydrogen atom, a C₁₋₃ alkyl group, or a halogen-substituted C₁₋₃ alkyl group, and preferably a trifluoromethyl group.

In one or more embodiments, an example of R² in Formula (I) above is a hydrogen atom.

In one or more embodiments, examples of R³ in Formula (I) above include a decahydroisoquinoline group, a *trans*-decahydroisoquinoline group, and a *cis*-decahydroisoquinoline group, and specific examples thereof are as follows.

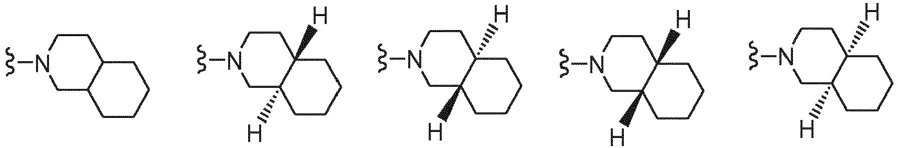

In one or more embodiments, a compound according to the present disclosure is a compound represented by Formula (III) below, a prodrug thereof, or a pharmaceutically acceptable salt thereof.

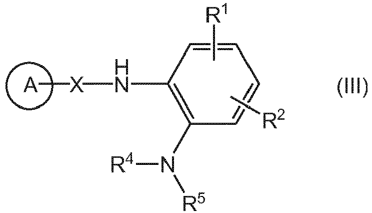

In one or more embodiments, a compound according to the present disclosure is a compound represented by Formulae (IVa) to (IVe) below, a prodrug thereof, or a pharmaceutically acceptable salt thereof.

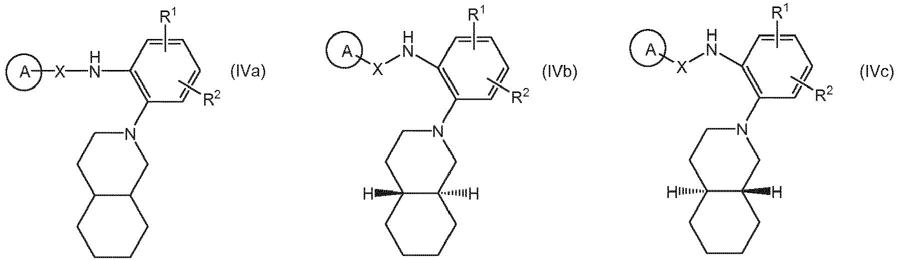

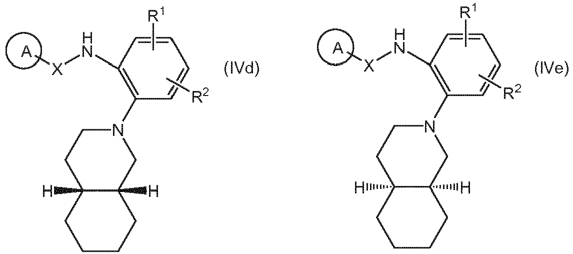

In one or more embodiments, X in Formulae (IVa) to (IVe) represents -C(=O)-, -C(=S)-, or -SO₂-, and preferably -C(=S)- or -SO₂-. From the viewpoint of inducing programmed cell death in cancer cells, in one or more embodiments, X in Formula (IVb) represents -SO₂-. From the viewpoint of inducing programmed cell death in cancer cells, in one or more embodiments, X in Formula (IVc) represents -C(=O)- or -C(=S)-.

In one or more embodiments, examples of A in Formulae (IVa) to (IVe) above include 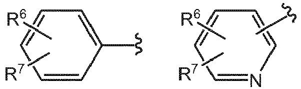 where R⁶ represents a hydrogen atom, a halogen atom, a hydroxyl group, or a substituted or unsubstituted C₁₋₆ alkyl group, and R⁷ represents a hydrogen atom.

In one or more embodiments, preferably, examples of A in Formulae (IVa) to (IVe) above include 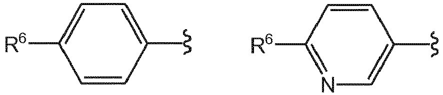 where R⁶ represents a hydrogen atom, a halogen atom, a hydroxyl group, or a substituted or unsubstituted C₁₋₆ alkyl group, and, in one or more embodiments, R⁶ represents a hydrogen atom, a halogen atom, or a hydroxyl group, and preferably a fluorine atom or a bromine atom.

In one or more embodiments, more preferably, examples of A in Formulae (IVa) to (IVe) above include 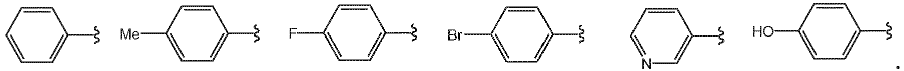 Even more preferably, examples thereof include

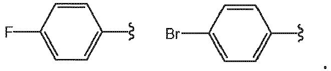

In one or more embodiments, a compound according to the present disclosure is a compound represented by Formulae (Va) to (VIIIe) below, a prodrug thereof, or a pharmaceutically acceptable salt thereof.

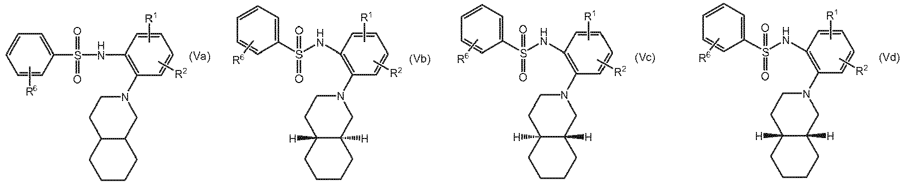

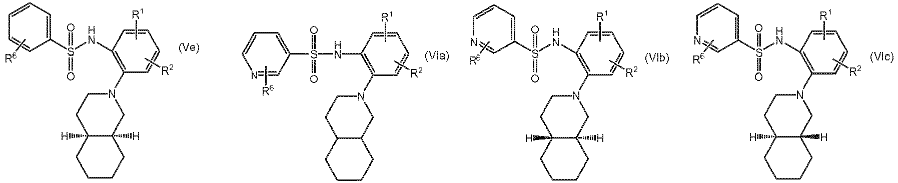

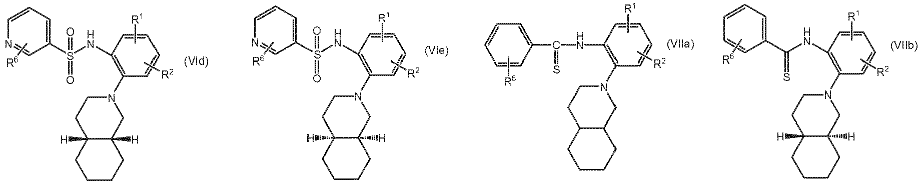

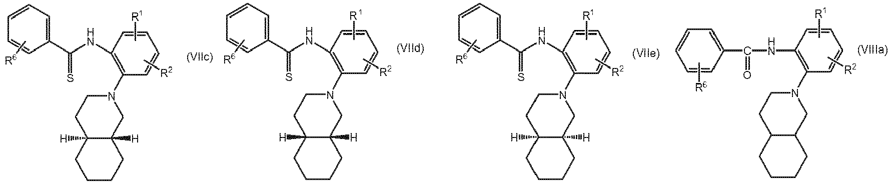

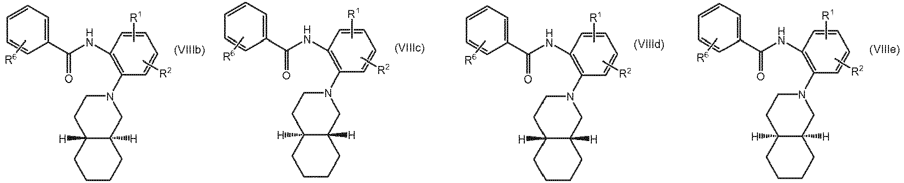

In one or more embodiments, a compound according to the present disclosure is a compound represented by Formulae (IXa) to (XIIe) below, a prodrug thereof, or a pharmaceutically acceptable salt thereof.

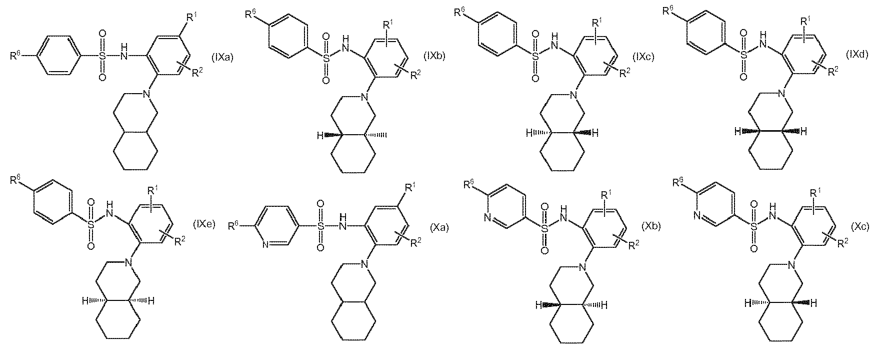

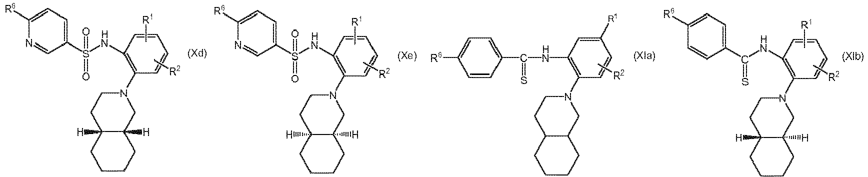

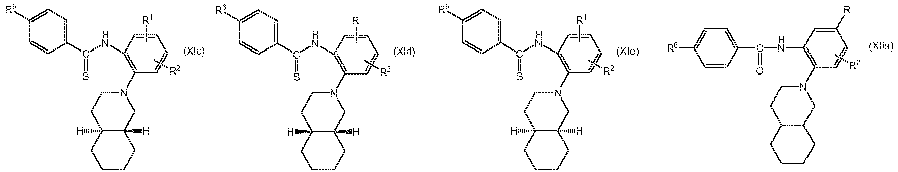

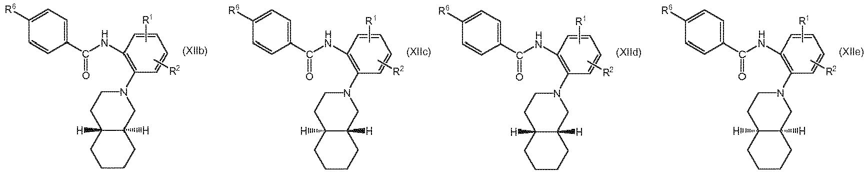

The description of Formula (I) above can be referred to for A and R¹ to R⁶ in Formulae (III) to (XIIe) above.

In Formulae (I) and (III) to (XIIe) above, from the viewpoint of inducing programmed cell death in cancer cells, R¹ is preferably a halogen tri-substituted C₁₋₃ alkyl group and more preferably a trifluoromethyl group, R² is preferably a hydrogen atom or a halogen atom and more preferably a hydrogen atom, R⁶ is preferably a hydrogen atom, a halogen atom, a nitro group, a hydroxy group, a C₁₋₃ alkoxy group, or a C₁₋₃ alkyl group, more preferably a hydrogen atom, a halogen atom, a methyl group, or a hydroxy group, and even more preferably a fluorine atom or a bromine atom.

In the present disclosure, in one or more embodiments, examples of the C₁₋₆ alkyl group include linear or branched alkyl groups such as a methyl group, an ethyl group, a 1-propyl group, a 2-propyl group, a 2-methyl-1-propyl group, a 2-methyl-2-propyl group, a 1-butyl group, a 2-butyl group, a 1-pentyl group, a 2-pentyl group, a 3-pentyl group, a 2-methyl-1-butyl group, a 3-methyl-1-butyl group, a 2-methyl-2-butyl group, a 3-methyl-2-butyl group, a 2,2-dimethyl-1-propyl group, a 1-hexyl group, a 2-hexyl group, a 3-hexyl group, a 2-methyl-1-pentyl group, a 3-methyl-1-pentyl group, a 4-methyl-1-pentyl group, a 2-methyl-2-pentyl group, a 3-methyl-2-pentyl group, a 4-methyl-2-pentyl group, a 2-methyl-3-pentyl group, a 3-methyl-3-pentyl group, a 2,3-dimethyl-1-butyl group, a 3,3-dimethyl-1-butyl group, a 2,2-dimethyl-1-butyl group, a 2-ethyl-1-butyl group, a 3,3-dimethyl-2-butyl group, and a 2,3-dimethyl-2-butyl group, and cyclic alkyl groups such as cyclopropyl, cyclobutyl, cyclopentyl, and cyclohexyl.

In the present disclosure, in one or more embodiments, the C₂₋₆ alkenyl group refers to a linear or branched alkenyl group having 2 to 6 carbon atoms, and specific examples thereof include a vinyl group, an allyl group, a 1-propenyl group, a 2-propenyl group, a 1-butenyl group, a 2-butenyl group, a 3-butenyl group, a pentenyl group, and a hexenyl group.

In the present disclosure, in one or more embodiments, the C₂₋₆ alkynyl group refers to a linear or branched alkynyl group having 2 to 6 carbon atoms, and specific examples thereof include an ethynyl group, a 1-propynyl group, a 2-propynyl group, a butynyl group, a pentynyl group, and a hexynyl group.

In the present disclosure, in one or more embodiments, the C₁₋₆ alkoxy group refers to an oxy group to which the C₁₋₆ alkyl group defined above is bonded, and specific examples include a methoxy group, an ethoxy group, a 1-propyloxy group, a 2-propyloxy group, a 2-methyl-1-propyloxy group, a 2-methyl-2-propyloxy group, a 1-butyloxy group, a 2-butyloxy group, a 1-pentyloxy group, a 2-pentyloxy group, a 3-pentyloxy group, a 2-methyl-1-butyloxy group, a 3-methyl-1-butyloxy group, a 2-methyl-2-butyloxy group, a 3-methyl-2-butyloxy group, a 2,2-dimethyl-1-propyloxy group, a 1-hexyloxy group, a 2-hexyloxy group, a 3-hexyloxy group, a 2-methyl-1-pentyloxy group, a 3-methyl-1-pentyloxy group, a 4-methyl-1-pentyloxy group, a 2-methyl-2-pentyloxy group, a 3-methyl-2-pentyloxy group, a 4-methyl-2-pentyloxy group, a 2-methyl-3-pentyloxy group, a 3-methyl-3-pentyloxy group, a 2,3-dimethyl-1-butyloxy, a 3,3-dimethyl-1-butyloxy group, a 2,2-dimethyl-1-butyloxy group, a 2-ethyl-1-butyloxy group, a 3,3-dimethyl-2-butyloxy group, and a 2,3-dimethyl-2-butyloxy group.

In the present disclosure, in one or more embodiments, the C₁₋₆ alkylthio group refers to a thio group to which the C₁₋₆ alkyl group defined above is bonded, and specific examples thereof include a methylthio group, an ethylthio group, a 1-propylthio group, a 2-propylthio group, a butylthio group, and a pentylthio group.

In the present disclosure, in one or more embodiments, the C₁₋₆ alkyl sulfonyl group refers to a sulfonyl group to which the C₁₋₆ alkyl group defined above is bonded, and specific examples thereof include a methylsulfonyl group, an ethylsulfonyl group, a 1-propyl sulfonyl group, and a 2-propyl sulfonyl group.

In the present disclosure, in one or more embodiments, the C₁₋₆ alkoxycarbonyl group refers to a carbonyl group to which the C₁₋₆ alkyl group defined above is bonded, and specific examples thereof include a methoxycarbonyl group, an ethoxycarbonyl group, a 1-propyloxycarbonyl group, and a 2-propyloxycarbonyl group.

In the present disclosure, in one or more embodiments, the C₆₋₁₀ aryl group refers to an aromatic hydrocarbon cyclic group having 6 to 10 carbon atoms, and specific examples thereof include a phenyl group, a 1-naphthyl group, and a 2-naphthyl group.

In the present disclosure, a "heterocyclic ring" refers to a non-aromatic ring or an aromatic ring that contains 1 to 2 heteroatoms of the atoms constituting the ring, and may contain a double bond in the ring. In the present disclosure, a "heteroatom" refers to a sulfur atom, an oxygen atom, or a nitrogen atom. In the present disclosure, a heterocyclic ring may be a fused heterocyclic ring in which two or more rings are fused.

In the present disclosure, a "nitrogen-containing heterocyclic ring" refers to a non-aromatic ring or an aromatic ring that contains 1 to 2 nitrogen atoms of the atoms constituting the ring, and may contain a double bond in the ring. If a nitrogen-containing heterocyclic ring is a fused heterocyclic ring, it is sufficient that a nitrogen atom is present in at least one ring.

In the present disclosure, one or more substituents that are the same as or different from each other may be included, and in one or more embodiments, examples thereof include a halogen atom, a cyano group, a trifluoromethyl group, a nitro group, a hydroxy group, a methylenedioxy group, a C₁₋₆ alkyl group, a C₁₋₆ alkoxy group, a benzyloxy group, a C₁₋₆ alkanoyloxy group, an amino group, a mono C₁₋₆ alkylamino group, a di C₁₋₆ alkylamino group, a carbamoyl group, a C₁₋₆ alkylaminocarbonyl group, a di C₁₋₆ alkylaminocarbonyl group, a carboxyl group, a C₁₋₆ alkoxycarbonyl group, a C₁₋₆ alkylthio group, a C₁₋₆ alkylsulfinyl group, a C₁₋₆ alkylsulfonyl group, a C₁₋₆ alkanoylamino group, and a C₁₋₆ alkyl sulfonamide group. In the present disclosure, in one or more embodiments, examples of a halogen atom include a fluorine atom, a chlorine atom, a bromine atom, and an iodine atom.

Also, if a compound according to the present disclosure includes an asymmetric carbon atom and/or a stereoisomer, in one or more embodiments, the compound is a mixture of stereoisomers or an isolated compound. In one or more embodiments in which a stereoisomer is not particularly limited, examples of a stereoisomer include *cis-trans* isomers.

In one or more embodiments, a "prodrug" in the present disclosure is soon hydrolyzed in a living body and reproduces a compound represented by Formula (I), and examples thereof include, if a compound has a carboxyl group, a compound whose carboxyl group has changed to an alkoxycarbonyl group, a compound whose carboxyl group has changed to an alkylthio carbonyl group, and a compound whose carboxyl group has changed to an alkylaminocarbonyl group. Also, examples thereof include, if a compound has an amino group, a compound whose amino group is substituted with an alkanoyl group to form an alkanoylamino group, a compound whose amino group is substituted with an alkoxycarbonyl group to form an alkoxycarbonylamino group, a compound whose amino group is substituted to form an acyloxymethylamino group, and a compound whose amino group is substituted to form a hydroxylamine. Also, examples thereof include, if a compound has a hydroxy group, a compound whose hydroxy group is substituted with the acyl group to form an acyloxy group, a compound whose hydroxy group is substituted to form a phosphoric acid ester, and a compound whose hydroxy group is substituted to form an acyloxymethyloxy group. An example of an alkyl portion of a group used to form these prodrugs is the alkyl group, and the alkyl group may be substituted (by an alkoxy group having 1 to 6 carbon atoms, for example). In one or more embodiments, examples of a compound whose carboxyl group has changed to an alkoxycarbonyl group include a lower (e.g., 1 to 6 carbon atoms) alkoxycarbonyl such as methoxycarbonyl and ethoxycarbonyl, and a lower (e.g., 1 to 6 carbon atoms) alkoxycarbonyl obtained through substitution with an alkoxy group, such as methoxymethoxycarbonyl, ethoxymethoxycarbonyl, 2-methoxyethoxycarbonyl, 2-methoxyethoxymethoxycarbonyl, and pivaloyloxymethoxycarbonyl.

In the present disclosure, a "pharmaceutically acceptable salt" refers to a pharmaceutically, pharmacologically, and/or medicinally acceptable salt, and examples thereof include inorganic acid salts, organic acid salts, inorganic base salts, organic base salts, and acidic or basic amino acid salts.

Preferred examples of the inorganic acid salts include hydrochloride, hydrobromide, sulfate, nitrate, and phosphate, and preferred examples of the organic acid salts include acetate, succinate, fumarate, maleate, tartrate, citrate, lactate, stearate, benzoate, methanesulfonate, and *p*-toluenesulfonate.

Preferred examples of the inorganic base salts include alkali metal salts such as sodium salts and potassium salts, alkaline earth salts such as calcium salts and magnesium salts, aluminum salts, and ammonium salts. Preferred examples of the organic base salts include diethylamine salts, diethanolamine salts, meglumine salts, and N', N-dibenzylethylenediamine salts.

Preferred examples of the acidic amino acid salts include aspartate and glutamate. Preferred examples of the basic amino acid salts include arginine salts, lysine salts, and ornithine salts.

In the present disclosure, a "salt of a compound" may include a hydrate that can be formed as a result of a compound being left in the air and absorbing moisture. Also, in the present disclosure, a "salt of a compound" may include a solvate that can be formed as a result of a compound absorbing a certain type of solvent.

In one or more embodiments that are not limited, examples of a compound according to the present disclosure include compounds represented by the chemical formulae below.

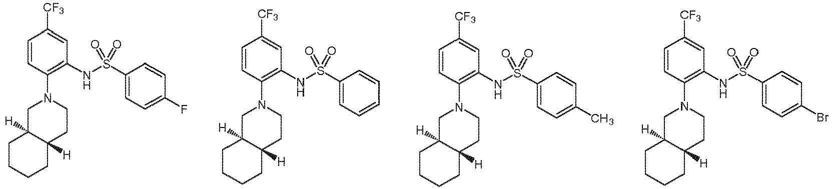

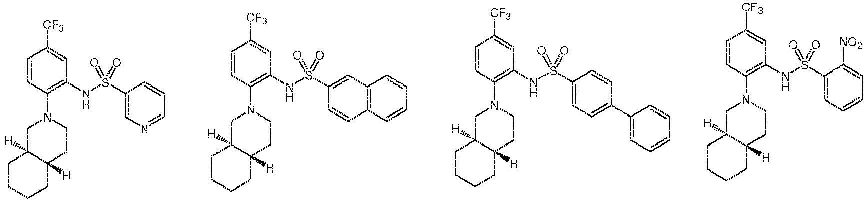

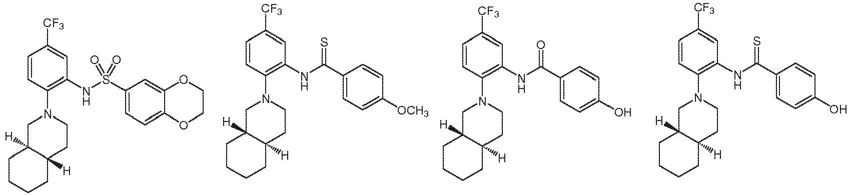

A compound according to the present disclosure can be used in an induction method according to the present disclosure (a method for inducing programmed cell death in cancer cells), and can be used as an active component of a pharmaceutical composition (a pharmaceutical composition for inducing programmed cell death in cancer cells) according to the present disclosure. Also, similarly to a pharmaceutical composition according to the present disclosure, a compound according to the present disclosure can be used in a method for preventing, improving, suppressing progression of, and/or treating cancers.

Thus, the present disclosure may relate to the following one or more embodiments.
[C1] Use of a compound (a compound according to the present disclosure) that enhances production of NO in cancer cells, in a method for preventing, improving, suppressing progression of, and/or treating cancers.
[C2] Use of a compound (a compound according to the present disclosure) that enhances production of NO in cancer cells, and an agent that reduces production of NADPH in cancer cells, in a method for preventing, improving, suppressing progression of, and/or treating cancers.
[C2-1] Use of a compound (a compound according to the present disclosure) that enhances production of NO in cancer cells, and at least one inhibition selected from the group consisting of inhibition of glycolysis, inhibition of a pentose phosphate pathway, inhibition of a malate dehydrogenase, inhibition of an isocitrate dehydrogenase, and inhibition of an amino acid transporter, in a method for preventing, improving, suppressing progression of, and/or treating cancers.
[C2-2] Use of a compound (a compound according to the present disclosure) that enhances production of NO in cancer cells, and an inhibitor of glycolysis, in a method for preventing, improving, suppressing progression of, and/or treating cancers.
[C2-3] Use of a compound (a compound according to the present disclosure) that enhances production of NO in cancer cells, and a substance having hexokinase inhibitory activity, in a method for preventing, improving, suppressing progression of, and/or treating cancers.
[C2-4] Use of a compound (a compound according to the present disclosure) that enhances production of NO in cancer cells, and a glucose derivative that cannot be metabolized through glycolysis or a glucose derivative that serves as an intermediate metabolite that cannot be metabolized through glycolysis, in a method for preventing, improving, suppressing progression of, and/or treating cancers.
[C2-5] Use of a compound (a compound according to the present disclosure) that enhances production of NO in cancer cells, and a substrate of a nitric oxide synthase and/or a substance that serves as a substrate of a nitric oxide synthase, in a method for preventing, improving, suppressing progression of, and/or treating cancers.
[C3] The use according to [C1] or [C2], in which enhancement of production of nitric oxide is enhancement of production of nitric oxide via a transmembrane protein.
[C4] The use according to any of [C1] to [C3], in which the cancer cells are cancer cells that produce ATP through glycolysis rather than oxidative phosphorylation.
[C5] The use according to any of [C1] to [C4], in which the cancer cells are cancer cells in which the Warburg effect is expressed.
[C6] Use of a pharmaceutical composition according to the present disclosure in prevention, amelioration, suppression of progression of, and/or treatment of cancers.
[C7] Use of a pharmaceutical composition according to the present disclosure, and an agent that reduces production of NADPH in cancer cells, in prevention, amelioration, suppression of progression of, and/or treatment of cancers.
[C7-1] Use of a pharmaceutical composition according to the present disclosure, and at least one inhibition selected from the group consisting of inhibition of glycolysis, inhibition of a pentose phosphate pathway, inhibition of a malate dehydrogenase, inhibition of an isocitrate dehydrogenase, and inhibition of an amino acid transporter, in prevention, amelioration, suppression of progression of, and/or treatment of cancers.
[C7-2] Use of a pharmaceutical composition according to the present disclosure, and an inhibitor of glycolysis, in prevention, amelioration, suppression of progression of, and/or treatment of cancers.
[C7-3] Use of a pharmaceutical composition according to the present disclosure, and a substance having hexokinase inhibitory activity, in prevention, amelioration, suppression of progression of, and/or treatment of cancers.
[C7-4] Use of a pharmaceutical composition according to the present disclosure, and a glucose derivative that cannot be metabolized through glycolysis or a glucose derivative that serves as an intermediate metabolite that cannot be metabolized through glycolysis, in prevention, amelioration, suppression of progression of, and/or treatment of cancers.
[C7-5] Use of a pharmaceutical composition according to the present disclosure, and a substrate of a nitric oxide synthase and/or a substance that serves as a substrate of a nitric oxide synthase, in prevention, amelioration, suppression of progression of, and/or treatment of cancers.
[C8] Use of a compound according to the present disclosure in manufacturing of a pharmaceutical composition for preventing, improving, suppressing progression of, and/or treating cancers.
[C9] Use of a pharmaceutical composition according to the present disclosure, and an agent that reduces production of NADPH in cancer cells, in manufacturing of a pharmaceutical composition for preventing, improving, suppressing progression of, and/or treating cancers.
   [C9-1] Use of a pharmaceutical composition according to the present disclosure, and at least one inhibition selected from the group consisting of inhibition of glycolysis, inhibition of a pentose phosphate pathway, inhibition of a malate dehydrogenase, inhibition of an isocitrate dehydrogenase, and inhibition of an amino acid transporter, in prevention, amelioration, suppression of progression of, and/or treatment of cancers, in manufacturing of a pharmaceutical composition for preventing, improving, suppressing progression of, and/or treating cancers.
   [C9-2] Use of a pharmaceutical composition according to the present disclosure, and an inhibitor of glycolysis, in manufacturing of a pharmaceutical composition for preventing, improving, suppressing progression of, and/or treating cancers.
   [C9-3] Use of a pharmaceutical composition according to the present disclosure, and a substance having hexokinase inhibitory activity, in manufacturing of a pharmaceutical composition for preventing, improving, suppressing progression of, and/or treating cancers.
   [C9-4] Use of a pharmaceutical composition according to the present disclosure, and a glucose derivative that cannot be metabolized through glycolysis or a glucose derivative that serves as an intermediate metabolite that cannot be metabolized through glycolysis, in manufacturing of a pharmaceutical composition for preventing, improving, suppressing progression of, and/or treating cancers.
   [C9-5] Use of a pharmaceutical composition according to the present disclosure, and a substrate of a nitric oxide synthase and/or a substance that serves as a substrate of a nitric oxide synthase, in manufacturing of a pharmaceutical composition for preventing, improving, suppressing progression of, and/or treating cancers.
[C10] A method for preventing, improving, suppressing progression of, and/or treating cancers, the method including administering, to a subject, a pharmaceutical composition according to the present disclosure.
[C11] The method according to [C10], further including reducing, in the cancer cells, production of NADPH in cancer cells.
[C12] The method according to [C10], further including administering, to a subject, at least one inhibition selected from the group consisting of inhibition of glycolysis, inhibition of a pentose phosphate pathway, inhibition of a malate dehydrogenase, inhibition of an isocitrate dehydrogenase, and inhibition of an amino acid transporter.
[C13] The method according to [C10], further including administering, to a subject, an inhibitor of glycolysis.
[C14] The method according to [C10], further including administering, to a subject, a substance having hexokinase inhibitory activity.
[C15] A method further including administering, to a subject, a glucose derivative that cannot be metabolized through glycolysis or a glucose derivative that serves as an intermediate metabolite that cannot be metabolized through glycolysis.
[C16] A method further including administering, to a subject, a substrate of a nitric oxide synthase and/or a substance that serves as a substrate of a nitric oxide synthase.

### Pharmaceutical composition for diseases attributable to a decrease in production of NO in cells

In one or more embodiments, a compound according to the present disclosure may exhibit the effect of activating nitric oxide synthases (NOSs) and the like, inducing production of NO, and enhancing production of NO in normal cells other than cancer cells. Thus, in one or more embodiments, a compound according to the present disclosure can be used to prevent, ameliorate, suppress progression of, and/or treat diseases attributable to a decrease in the production of NO in cells, and can be used as an active component of a pharmaceutical composition for these diseases.

As another aspect, the present disclosure relates to a pharmaceutical composition for preventing, improving, suppressing progression of, and/or treating diseases attributable to a decrease in the production of NO in cells, the pharmaceutical composition containing, as an active component, a compound represented by Formula (I) or (III), a prodrug thereof, or a pharmaceutically acceptable salt thereof.

As another aspect, the present disclosure relates to a method for preventing, improving, suppressing progression of, and/or treating diseases attributable to a decrease in production of NO in cells, the method including administering a compound represented by Formula (I) or (III), a prodrug thereof, or a pharmaceutically acceptable salt thereof, to a subject that requires the compound represented by Formula (I) or (III), the prodrug thereof, or the pharmaceutically acceptable salt thereof.

In one or more embodiments, examples of diseases attributable to a decrease in the production of NO in cells include cardiovascular diseases and the like resulting from a decrease in the production of NO in cells. In one or more embodiments, examples of diseases attributable to a decrease in the production of NO include hypertension, pulmonary hypertension, respiratory failure, heart failure, arteriosclerosis, hyperlipidemia, and coronary artery spasm. "Cells" in the present disclosure refer to human cells and cells of an animal other than a human.

### Pharmaceutical composition for lifestyle diseases and/or metabolic syndrome

In one or more embodiments, a compound according to the present disclosure may exhibit the effect of enhancing production of NO in normal cells, and thus may exhibit the effect of facilitating consumption of carbohydrates in the body. Thus, as another aspect, the present disclosure relates to a pharmaceutical composition for preventing, improving, suppressing progression of, and/or treating lifestyle diseases and/or metabolic syndrome, the pharmaceutical composition containing, as an active component, a compound represented by Formula (I) or (III), a prodrug thereof, or a pharmaceutically acceptable salt thereof.

As another aspect, the present disclosure relates to a method for preventing, improving, suppressing progression of, and/or treating lifestyle diseases and/or metabolic syndrome, the method being administering a compound represented by Formula (I) or (III), a prodrug thereof, or a pharmaceutically acceptable salt thereof, to a subject that requires the compound represented by Formula (I) or (III), the prodrug thereof, or the pharmaceutically acceptable salt thereof.

### Examples

Hereinafter, although the present disclosure will be described in more detail by way of examples, but these examples are merely exemplary, and the present disclosure is not limited to these examples. Note that the entirety of references cited in the present disclosure is incorporated as a portion of the present disclosure.

### Production Example 1: Production of Compound 1

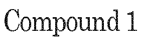

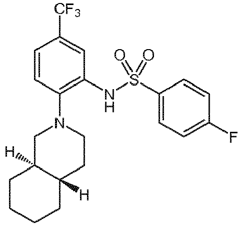

Compound 1 was synthesized as follows.

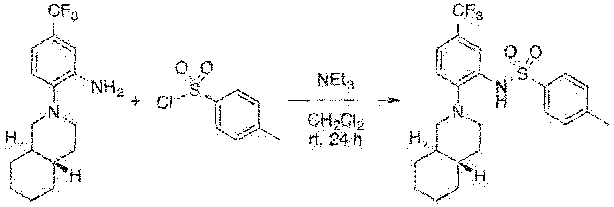

4-fluorobenzenesulfonyl chloride (2.90 g, 14.9 mmol, a commercial product), triethylamine (1.00 mL, 7.23 mmol, a commercial product), and N,N-dimethyl-4-aminopyridine (10 mg, 0.082 mmol, a commercial product) were successively added at room temperature to a dichloromethane solution (20 mL) containing 2-[(4aS,8aR)-octahydroisoquinolin-2(1H)-yl]-5-(trifluoromethyl)aniline (1.20 g, 4.03 mmol) synthesized using the method cited in a reference (PCT Int. Appl.(2009), WO2009119167 A1 20091001.), and then the resulting mixture was stirred for 48 hours. After water was added to this reaction mixture to stop the reaction, extraction was performed using ethyl acetate (x3), the extracted product was washed with a saturated saline solution, and then dried using anhydrous sodium sulfate. After the resulting mixture was filtered, the filtrate was concentrated under reduced pressure. Next, tetra-n-butylammonium fluoride (1.0 M in THF, 10 mL, 10 mmol) was added at room temperature to a tetrahydrofuran solution (20 mL) containing the obtained reaction crude product, and the resulting mixture was stirred for 12 hours. After the resulting mixture was concentrated under reduced pressure, the obtained reaction crude product was purified through silica gel column chromatography (Biotage, SNAP Ultra 100 g, hexane/ethyl acetate = 50/1 to 20/1) and then re-crystallized (ethyl acetate/hexane), and thus 4-fluoro-N-(2-((4aS,8aR)-octahydroisoquinolin-2(1H)-yl) -5-(trifluoromethyl)phenyl)benzenesulfonamide (647 mg, 1.42 mmol, 35.2%) (Compound 1) was obtained as colorless crystals.
TLC Rf0.44 (hexane/ethyl acetate = 10/1); ¹H NMR (CDCl₃, 400 MHz) δ 0.89-1.13 (m, 3H), 1.22-1.48 (m, 5H), 1.50-1.72 (m, 2H), 1.73-1.81 (m, 2H), 2.24 (dd, 1H, J = 10.8, 10.8 Hz), 2.37-2.42 (m, 1H), 2.50-2.62 (m, 2H), 7.09-7.16 (m, 3H), 7.26-7.30 (m, 1H), 7.82-7.88 (m, 3H), 7.98 (br s, 1H).

### Production Example 2: Production of Compound 2

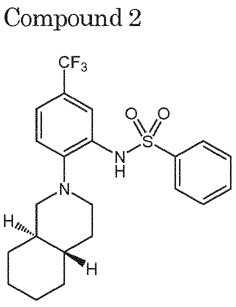

Compound 2 was synthesized as follows.

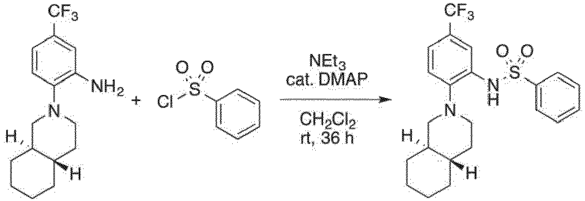

Benzenesulfonyl chloride (77 µL, 0.60 mmol, a commercial product), triethylamine (0.10 mL, 0.72 mmol, a commercial product), and N,N-dimethyl-4-aminopyridine (10 mg, 0.082 mmol, a commercial product) were successively added at room temperature to a dichloromethane solution (10 mL) containing 2-[(4aS,8aR)-octahydroisoquinolin-2(1H)-yl]-5-(trifluoromethyl)aniline (120 mg, 0.403 mmol) synthesized using the method cited in the reference (PCT Int. Appl.(2009), WO2009119167 A1 20091001.), and then the resulting mixture was stirred for 36 hours. After water was added to this reaction mixture to stop the reaction, extraction was performed using ethyl acetate (x3), the extracted product was washed with a saturated saline solution, and then dried using anhydrous sodium sulfate. After the resulting mixture was filtered, the filtrate was concentrated under reduced pressure. The obtained reaction crude product was purified through silica gel column chromatography (KANTO CHEMICAL CO., INC., neutral, spherical, 10 g, hexane/ethyl acetate = 20/1) and then re-crystallized (ethyl acetate/hexane), and thus N-(2-((4aS,8aR)-octahydroisoquinolin-2(1H)-yl)-5-(trifluoromethyl)phenyl)benzenesulfo namide (146 mg, 0.33 mmol, 83.1%) (Compound 2) was obtained as colorless crystals. TLC Rf0.19 (hexane/ethyl acetate = 20/1); ¹H NMR (CDCl₃, 400 MHz) δ 0.78-1.12 (m, 3H), 1.13-1.48 (m, 5H), 1.55-1.80 (m, 4H), 2.20 (dd, 1H, J = 10.4,10.4 Hz), 2.31-2.38 (m, 1H), 2.49-2.59 (m, 2H), 7.12 (d, 1H, J = 8.4 Hz), 7.26 (dd, 1H, J = 8.4, 1.6 Hz), 7.42-7.47 (m, 2H), 7.51-7.57 (m, 1H), 7.81-7.85 (m, 2H), 7.87 (dd, 1H, J = 1.6 Hz), 7.97 (br s, 1H).

### Production Example 3: Production of Compound 3

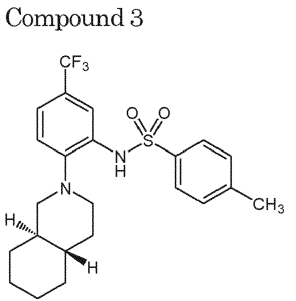

Compound 3 was synthesized as follows.

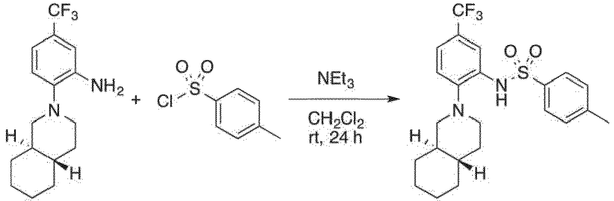

4-toluenesulfonyl chloride (460 mg, 2.41 mmol, a commercial product) and triethylamine (0.33 mL, 2.39 mmol, a commercial product) were successively added at room temperature to a dichloromethane solution (5.0 mL) containing 2-[(4aS,8aR)-octahydroisoquinolin-2(1H)-yl]-5-(trifluoromethyl)aniline (600 mg, 2.03 mmol) synthesized using the method cited in the reference (PCT Int. Appl.(2009), WO2009119167 A1 20091001.), and then the resulting mixture was stirred for 24 hours. After water was added to this reaction mixture to stop the reaction, extraction was performed using ethyl acetate (x3), the extracted product was washed with a saturated saline solution, and then dried using anhydrous sodium sulfate. After the resulting mixture was filtered, the filtrate was concentrated under reduced pressure. The obtained reaction crude product was purified through silica gel column chromatography (KANTO CHEMICAL CO., INC., neutral, spherical, 20 g, hexane/ethyl acetate = 50/1 to 20/1) and then re-crystallized (ethyl acetate/hexane), and thus
N-(2-((4aS,8aR)-octahydroisoquinolin-2(1H)-yl)-5-(trifluoromethyl)phenyl)-4-toluenesul fonamide (180 mg, 0.397 mmol, 19.9%) (Compound 3) was obtained as colorless crystals. TLC Rf0.43 (hexane/ethyl acetate = 10/1); ¹H NMR (CDCl₃, 400 MHz) δ 0.88-1.14 (m, 3H), 1.24-1.48 (m, 5H), 1.50-1.82 (m, 4H), 2.20 (dd, 1H, J = 10.8, 10.8 Hz), 2.35-2.41 (m, 4H), 2.54-2.59 (m, 2H), 7.12 (d, 1H, J = 8.0 Hz), 7.22-7.26 (m, 3H), 7.70-7.73 (AA'BB', 2H), 7.86 (d, 1H, J = 2.0 Hz), 7.95 (br s, 1H).

### Production Example 4: Production of Compound 4

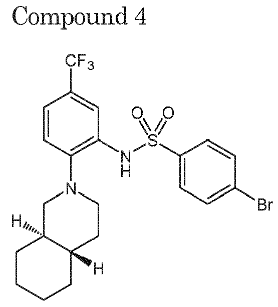

Compound 4 was synthesized as follows.

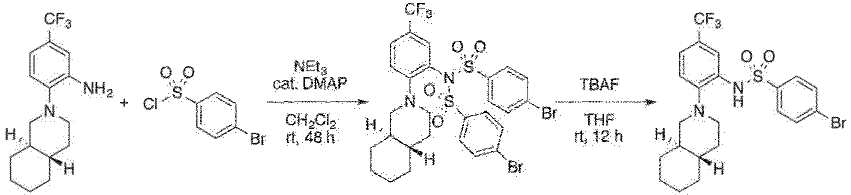

4-bromobenzenesulfonyl chloride (310 mg, 1.21 mmol, a commercial product), triethylamine (0.20 mL, 1.45 mmol, a commercial product), and N,N-dimethyl-4-aminopyridine (10 mg, 0.082 mmol, a commercial product) were successively added at room temperature to a dichloromethane solution (5.0 mL) containing 2-[(4aS,8aR)-octahydroisoquinolin-2(1H)-yl]-5-(trifluoromethyl)aniline (120 mg, 0.403 mmol) synthesized using the method cited in the reference (PCT Int. Appl.(2009), WO2009119167 A1 20091001.), and then the resulting mixture was stirred for 48 hours. After water was added to this reaction mixture to stop the reaction, extraction was performed using ethyl acetate (x3), the extracted product was washed with a saturated saline solution, and then dried using anhydrous sodium sulfate. After the resulting mixture was filtered, the filtrate was concentrated under reduced pressure. As a result of reprecipitating the obtained reaction crude product (ethyl acetate/hexane), a bis(sulfonyl)imide intermediate (232 mg, 0.315 mmol, 78.4%) was obtained.

Next, tetra-n-butylammonium fluoride (1.0 M in THF, 1.0 mL, 1.0 mmol) was added at room temperature to a tetrahydrofuran solution (20 mL) containing the obtained bis(sulfonyl)imide intermediate (152 mg, 0.207 mmol), and the resulting mixture was stirred for 12 hours. After the resulting mixture was concentrated under reduced pressure, the obtained reaction crude product was purified through silica gel column chromatography (KANTO CHEMICAL CO., INC., neutral, spherical, 10 g, hexane/ethyl acetate = 20/1 to 10/1) and then re-crystallized (ethyl acetate/hexane), and thus 4-bromo-N-(2-((4aS,8aR)-octahydroisoquinolin-2(1H)-yl)-5-(trifluoromethyl)phenyl)benzenesulfonamide (101 mg, 0.195 mmol, 94.4%) (Compound 4) was obtained as colorless crystals.
TLC Rf0.23 (hexane/ethyl acetate = 20/1); ¹H NMR (CDCl₃, 400 MHz) δ 0.88-1.12 (m, 3H), 1.32-1.50 (m, 5H), 1.61-1.73 (m, 2H), 1.73-1.81 (m, 2H), 2.23 (dd, 1H, J = 10.8,10.8 Hz), 2.32-2.40 (m, 1H), 2.50-2.62 (m, 2H), 7.15 (d, 1H, J = 8.4 Hz), 7.28 (dd, 1H, J = 8.4, 1.6 Hz), 7.56-7.61 (AA'BB', 2H), 7.67-7.70 (AA'BB', 2H), 7.84 (d, 1H, J = 1.6 Hz), 7.99 (br s, 1H).

### Production Example 5: Production of Compound 5

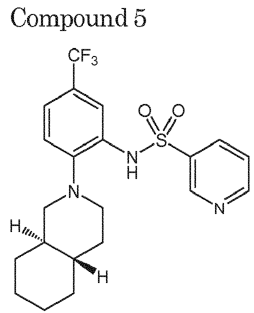

Compound 5 was synthesized as follows.

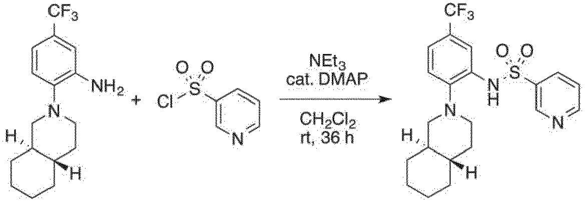

3-pyridinesulfonyl chloride hydrochloride (130 mg, 0.607 mmol, a commercial product), triethylamine (0.40 mL, 2.89 mmol, a commercial product), and N,N-dimethyl-4-aminopyridine (10 mg, 0.082 mmol, a commercial product) were successively added at room temperature to a dichloromethane solution (5.0 mL) containing 2-[(4aS,8aR)-octahydroisoquinolin-2(1H)-yl]-5-(trifluoromethyl)aniline (120 mg, 0.402 mmol) synthesized using the method cited in the reference (PCT Int. Appl.(2009), WO2009119167 A1 20091001.), and then the resulting mixture was stirred for 48 hours. After water was added to this reaction mixture to stop the reaction, extraction was performed using ethyl acetate (x3), the extracted product was washed with a saturated saline solution, and then dried using anhydrous sodium sulfate. After the resulting mixture was filtered, the filtrate was concentrated under reduced pressure. The obtained reaction crude product was purified through silica gel column chromatography (KANTO CHEMICAL CO., INC., neutral, spherical, 10 g, hexane/ethyl acetate = 5/1 to 1/1) and then purified through preparative TLC again (ethyl acetate/hexane = 1/1), and thus
N-(2-((4aS,8aR)-octahydroisoquinolin-2(1H)-yl)-5-(trifluoromethyl)phenyl)-3-pyridine-s ulfonamide (22.8 mg, 0.0519 mmol, 12.9%) (Compound 5) was obtained as colorless crystals.
TLC Rf0.21 (hexane/ethyl acetate = 2/1); ¹H NMR (CDCl₃, 400 MHz) δ 0.87-1.13 (m, 3H), 1.22-1.51 (m, 5H), 1.52-1.81 (m, 4H), 2.24 (dd, 1H, J = 10.8 10.8 Hz), 2.36-2.42 (m, 1H), 2.51-2.63 (m, 2H), 7.16 (d, 1H, J = 8.4 Hz), 7.30 (dd, 1H, J = 8.4, 1.6 Hz), 7.41 (ddd, 1H, J = 8.0, 4.8, 0.8 Hz), 7.85 (d, 1H, J = 1.6 Hz), 8.06-8.13 (m, 2H), 8.76 (dd, 1H, J = 4.8, 1.2 Hz), 9.04 (dd, 1H, J = 2.4, 0.8 Hz).

### Production Example 6: Production of Compound 6

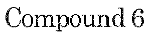

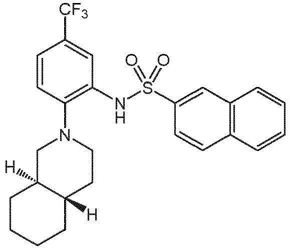

Compound 6 was synthesized as follows.

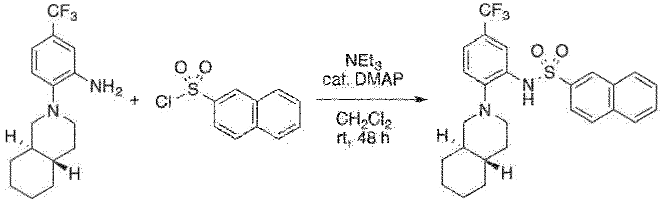

4-toluenesulfonyl chloride (140 mg, 0.617 mmol, a commercial product), triethylamine (0.10 mL, 0.72 mmol, a commercial product), and N,N-dimethyl-4-aminopyridine (10 mg, 0.082 mmol, a commercial product) were successively added at room temperature to a dichloromethane solution (5.0 mL) containing 2-[(4aS,8aR)-octahydroisoquinolin-2(1H)-yl]-5-(trifluoromethyl)aniline (120 mg, 0.402 mmol) synthesized using the method cited in the reference (PCT Int. Appl.(2009), WO2009119167 A1 20091001.), and then the resulting mixture was stirred for 48 hours. After water was added to this reaction mixture to stop the reaction, extraction was performed using ethyl acetate (x3), the extracted product was washed with a saturated saline solution, and then dried using anhydrous sodium sulfate. After the resulting mixture was filtered, the filtrate was concentrated under reduced pressure. The obtained reaction crude product was purified through silica gel column chromatography (KANTO CHEMICAL CO., INC., neutral, spherical, 20 g, hexane/ethyl acetate = 50/1 to 20/1) and then re-crystallized (ethyl acetate/hexane), and thus
N-(2-((4aS,8aR)-octahydroisoquinolin-2(1H)-yl)-5-(trifluoromethyl)phenyl)-2-naphthale nesulfonamide (50.6 mg, 0.103 mmol, 25.7%) (Compound 6) was obtained as colorless crystals.
TLC Rf0.34 (hexane/ethyl acetate = 10/1); ¹H NMR (CDCl₃, 400 MHz) δ 0.82-1.12 (m, 3H), 1.23-1.80 (m, 9H), 2.14 (dd, 1H, J = 11.2,11.2 Hz), 2.24-2.30 (m, 1H), 2.51-2.56 (m, 2H), 7.08 (d, 1H, J = 8.4 Hz), 7.20-7.24 (m, 1H), 7.57-7.65 (m, 2H), 7.75 (dd, 1H, J = 8.6, 1.6 Hz), 7.85-7.93 (m, 3H), 7.97 (d, 1H, J = 2.0 Hz), 8.08 (br s, 1H), 8.45 (s, 1H).

### Production Example 7: Production of Compound 7

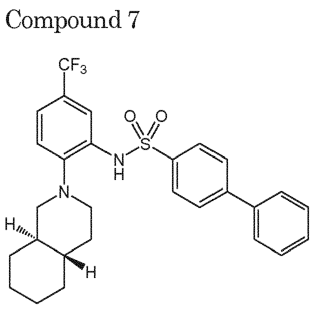

Compound 7 was synthesized with reference to the above-described synthesis method.

### Production Example 8: Production of Compound 8

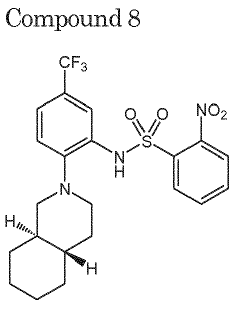

Compound 8 was synthesized with reference to the above-described synthesis method.

### Production Example 9: Production of Compound 9

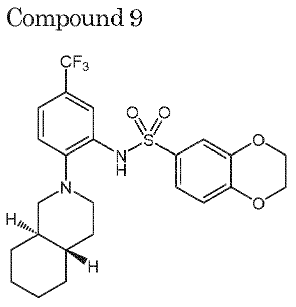

Compound 9 was synthesized with reference to the above-described synthesis method.

### Production Example 10: Production of Compound 10

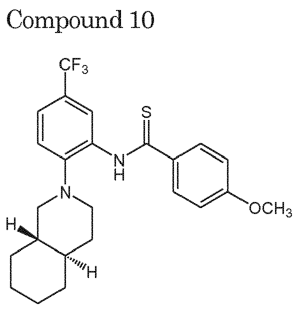

Compound 10 was synthesized with reference to the above-described synthesis method.

### Production Example 11: Production of Compound 11

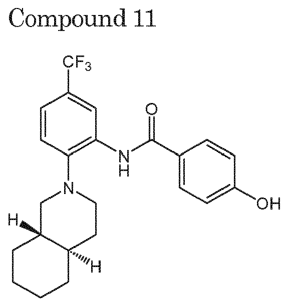

Compound 11 was synthesized with reference to the above-described synthesis method.

### Production Example 12: Production of Compound 12

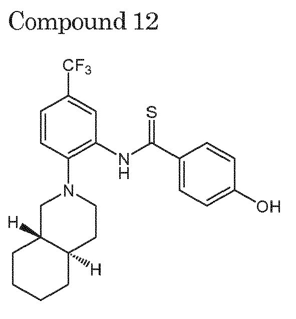

Compound 12 was synthesized with reference to the above-described synthesis method.

### Induction of production of NO in cancer cells

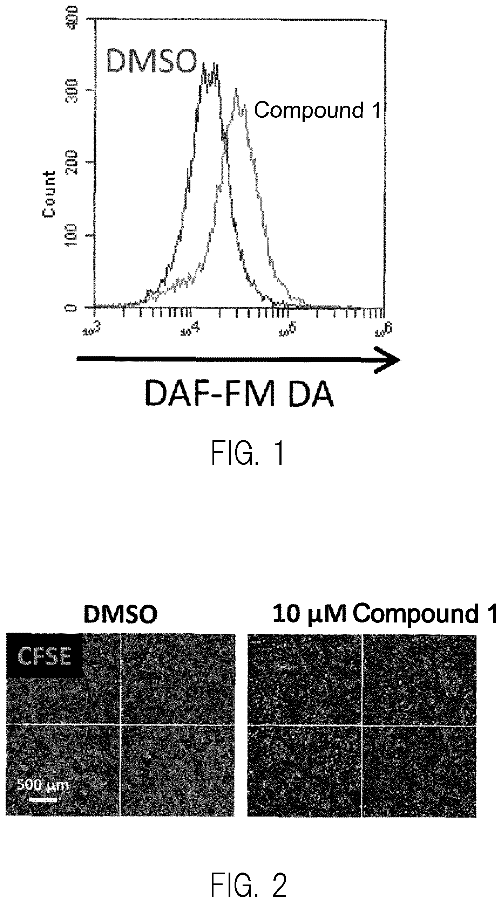

The amount of nitric oxide (NO) produced in cancer cells was measured using a nitric oxide detection indicator. Specifically, Compound 1 prepared as described above was added to cancer cells that were to be cultured under the following conditions such that the concentration of Compound 1 was 10 µM, and the cancer cells were cultured for 10 hours, then flow cytometry was performed while the NO detection indicator was in contact therewith, and fluorescence intensity and cell number distribution were measured. The results are shown in FIG. 1.
Cancer cells: Human cervical cancer cell line HeLa
Nutrient sources of culture medium: MEM (No Glucose) containing 0.1% Galactose and 5% FBS
Culture time after Compound 1 or DMSO was added: 10 hours at 37°C
Nitric oxide detection indicator: Diaminofluorescein-FM diacetate (DAF-FM DA)

As shown in FIG. 1, it was confirmed that, when Compound 1 was added, the production of NO in cells was enhanced.

### Antitumor activity

The antitumor effect on cancer cells was confirmed through measurement of the number of living cells using two methods.

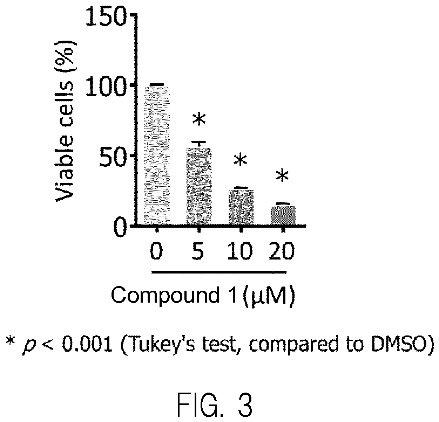

Specifically, Compound 1 prepared as described above was added to cancer cells that were to be cultured under the following conditions such that the concentration of Compound 1 was 5, 10, or 20 µM, cancer cells were cultured for 4 days, and then an effect of Compound 1 on cancer cells was confirmed through fluorescence staining of living cells using CFSE-DA (FIG. 2) and WST-8 assays (FIG. 3).
Cancer cells: Human cervical cancer cell line HeLa
Nutrient sources of culture medium: MEM containing 5% FBS
Culture time after Compound 1 or DMSO was added: 4 days at 37°C
CFSE: 5- or 6-(N-Succinimidyloxycarbonyl)fluorescein 3',6'-diacetate

As shown in FIGS. 2 and 3, Compound 1 exhibited an antitumor effect of reducing the survival rate of cancer cells.

Antitumor activity is programmed cell death
It was confirmed that the above-described antitumor effect was accompanied with the activation of Caspase-3, in other works, was programmed cell death.

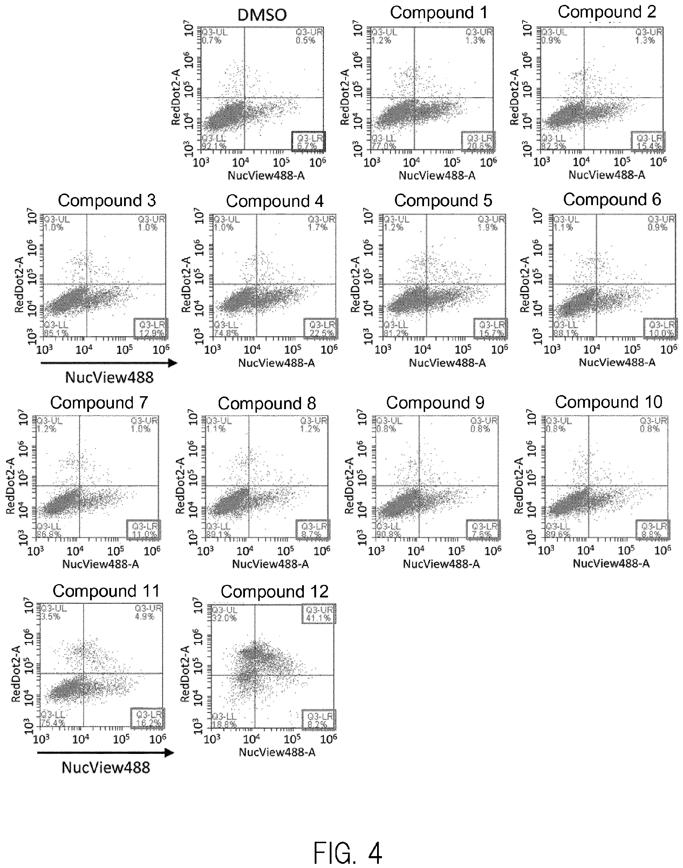

That is, Compounds 1 to 12 prepared as described above were added to cancer cells that were to be cultured under the following conditions such that the concentration of a compound was 10 µM, and the cancer cells were cultured for 16 hours, flow cytometry was then performed while a caspase 3 substrate and a membrane impermeable DNA stain were in contact therewith, and thus two-color dot plots were created. The results are shown in FIG. 4.
Cancer cells: Human cervical cancer cell line HeLa
Nutrient sources of culture medium: MEM (No Glucose) containing 0.1% Galactose and 5% FBS
Culture time after Compound 1 or DMSO was added: 16 hours at 37°C
Caspase 3 substrate: NucView (trademark, Biotium) 488
Membrane impermeable DNA stain: RedDot (trademark, Biotium) 2

As shown in FIG. 4, with cancer cells that were in contact with Compounds 1 to 12, the ratio of cells with RedDot (trademark) 2 negative and NucView (trademark) 488 positive at the bottom right increased to a value higher than 6.7%. For example, Compound 1 was 20.6%, Compound 2 was 15.4%, Compound 3 was 12.9%, Compound 4 was 22.5%, Compound 5 was 15.7%, and Compound 11 was 16.2%. Thus, Compounds 1 to 12 induced programmed cell death in cancer cells.

Induced programmed cell death is NO-dependent
It was confirmed that the activity of Caspase-3 above was NO-dependent, using two types of methods in which an NO scavenger and an NO synthesis inhibitor were used.

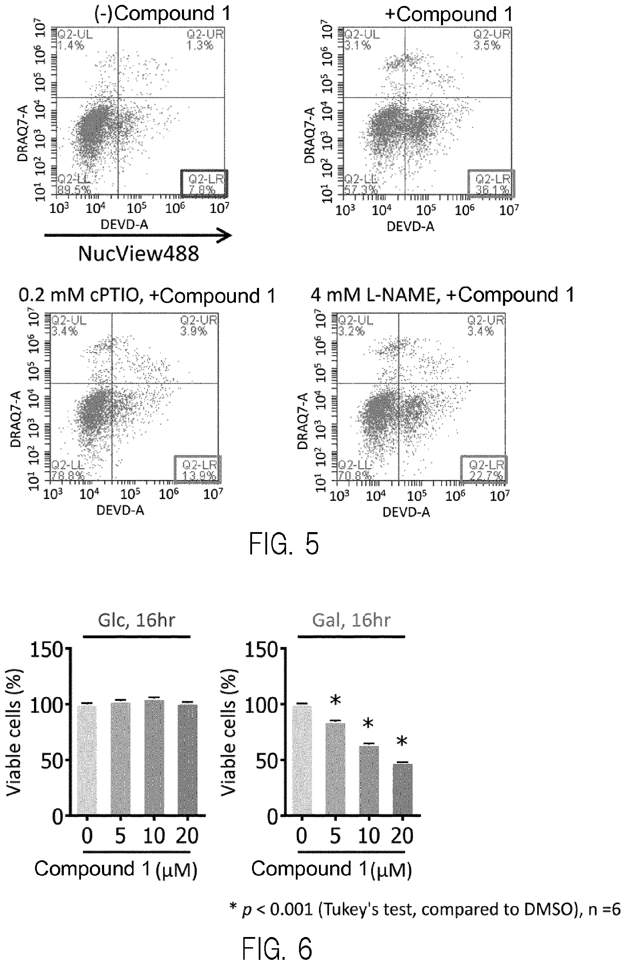

That is, first, the NO scavenger (0.2 mM) or NO synthesis inhibitor (4 mM) described above was added to cancer cells that were to be cultured under the following conditions, and the cancer cells were cultured for 1 hour, Compound 1 prepared as described above was added thereto such that the concentration of Compound 1 was 10 µM, the cancer cells were cultured for 15 hours, flow cytometry was then performed while a caspase 3 substrate and a membrane impermeable DNA stain were in contact therewith, and thus two-color dot plots were created. The results are shown in FIG. 5.
Cancer cells: Human cervical cancer cell line HeLa
Nutrient sources of culture medium: MEM (No Glucose) containing 0.1% Galactose and 5% FBS
Culture time after NO scavenger and NO synthesis inhibitor were added: 1 hour at 37°C
NO scavenger: Carboxy-PTIO (cPTIO)
NO synthesis inhibitor: L-NAME (NO synthatase inhibitor)
Culture time after Compound 1 or DMSO was added: 15 hours
Caspase 3 substrate: NucView (trademark, Biotium) 488
Membrane impermeable DNA stain: DRAQ7 (trademark, BioStatus)

As shown in FIG. 5, with cancer cells that were in contact with Compound 1, the ratio of cells with DRAQ7 (trademark) negative and NucView (trademark) 488 positive on the bottom right increased from 7.8% to 36.1%, but the cell ratio was reduced to 13.9% under the presence of cPTIO and 22.7% under the presence of L-NAME. Thus, programmed cell death induced by Compound 1 was NO-dependent.

That is, from the results of the above-described experiment, Compound 1 enhanced production of NO in cancer cells and induced NO-dependent programmed cell death in cancer cells.

Programmed cell death is facilitated through glucose starvation
It was confirmed that programmed cell death of cancer cells by Compound 1 was facilitated under glucose starvation.

Specifically, the nutrient source of a culture medium used when Compound 1 was applied to cancer cells was set to either glucose (Glc) or galactose (Gal), cancer cells were cultured for 16 hours, and the survival rate of cancer cells was measured using WST-8 assays. The results are shown in FIG. 6.
Cancer cells: Human cervical cancer cell line HeLa
Culture medium before Compound 1 was added: MEM containing 10% FBS
Culture medium after Compound 1 was added: MEM containing 0.1% Glucose and 5% FBS, or containing 0.1% Galactose and 5% FBS (No Glucose)
Culture time after Compound 1 was added: 16 hours at 37°C

As shown in FIG. 6, programmed cell death of cancer cells by Compound 1 was facilitated in a glucose depleted state.

### Antitumor activity

Compound 1 exhibited an antitumor effect on a plurality of cancer cell lines.

## Claims

1. A pharmaceutical composition for inducing programmed cell death in cancer cells, the pharmaceutical composition containing, as an active component,
a compound that enhances production of nitric oxide in cancer cells.

2. The pharmaceutical composition according to claim 1,
wherein enhancement of production of nitric oxide is enhancement of production of nitric oxide via a transmembrane protein.

3. The pharmaceutical composition according to claim 1 or 2,
wherein the cancer cells are cancer cells whose ATP production is dependent on glycolysis rather than oxidative phosphorylation.

4. The pharmaceutical composition according to any of claims 1 to 3,
wherein the cancer cells are cancer cells in which a Warburg effect is expressed.

5. The pharmaceutical composition according to any of claims 1 to 4,
wherein the compound is a compound represented by Formula (I) below, a prodrug thereof, or a pharmaceutically acceptable salt thereof, 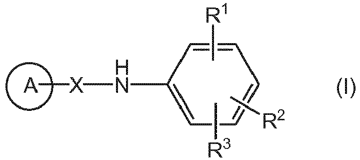
wherein A represents a substituted or unsubstituted monocyclic, bicyclic, or bicyclic fused aryl group or heteroaryl group,
X represents an oxygen atom, -C(=O)-, -C(=S)-, or -SO₂-,
R¹ represents a hydrogen atom, a substituted or unsubstituted C₁₋₆ alkyl group, a substituted or unsubstituted C₂₋₆ alkenyl group, a substituted or unsubstituted C₂₋₆ alkynyl group, a substituted or unsubstituted C₆₋₁₀ aryl group, a halogen atom, a nitro group, a cyano group, an azido group, a hydroxy group, a substituted or unsubstituted C₁₋₆ alkoxy group, a substituted or unsubstituted C₁₋₆ alkylthio group, a substituted or unsubstituted C₁₋₆ alkyl sulfonyl group, a carboxy group, a formyl group, a substituted or unsubstituted C₁₋₆ alkoxycarbonyl group, an acyl group, an acylamino group, or a sulfamoyl group,
R² represents a hydrogen atom, a substituted or unsubstituted C₁₋₆ alkyl group, or a halogen atom,
R³ represents a hydrogen atom, a substituted or unsubstituted C₁₋₆ alkyl group, a substituted or unsubstituted C₆₋₁₀ aryl group, a halogen atom, a hydroxy group, a substituted or unsubstituted C₁₋₆ alkoxy group, a substituted or unsubstituted C₁₋₆ alkylthio group, a substituted or unsubstituted nitrogen-containing heterocyclic ring, a substituted or unsubstituted fused aromatic heterocyclic ring, or is represented by Formula (II), 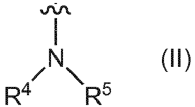
wherein R⁴ and R⁵ are the same as or different from each other, and represent a hydrogen atom, a substituted or unsubstituted C₁₋₆ alkyl group, a substituted or unsubstituted nitrogen-containing heterocyclic ring, a substituted or unsubstituted fused aromatic heterocyclic ring, an acyl group, or an acylamino group; or
R⁴ and R⁵ form a substituted or unsubstituted heterocyclic ring, or a substituted or unsubstituted fused aromatic heterocyclic ring, together with an adjacent nitrogen atom; or
R⁴ and R⁵ represent a substituted or unsubstituted cycloalkylidene amino group, or a substituted or unsubstituted aromatic ring fused cycloalkylidene group.

6. The pharmaceutical composition according to any of claims 1 to 5,
wherein the compound is a compound represented by Formula (III), a prodrug thereof, or a pharmaceutically acceptable salt thereof, 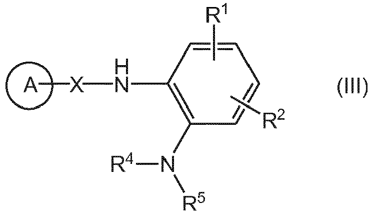
wherein A represents a substituted or unsubstituted monocyclic, bicyclic, or bicyclic fused aryl group or heteroaryl group,
X represents an oxygen atom, -C(=O)-, -C(=S)-, or -SO₂-,
R¹ represents a hydrogen atom, a substituted or unsubstituted C₁₋₆ alkyl group, a substituted or unsubstituted C₂₋₆ alkenyl group, a substituted or unsubstituted C₂₋₆ alkynyl group, a substituted or unsubstituted C₆₋₁₀ aryl group, a halogen atom, a nitro group, a cyano group, an azido group, a hydroxy group, a substituted or unsubstituted C₁₋₆ alkoxy group, a substituted or unsubstituted C₁₋₆ alkylthio group, a substituted or unsubstituted C₁₋₆ alkyl sulfonyl group, a carboxy group, a formyl group, a substituted or unsubstituted C₁₋₆ alkoxycarbonyl group, an acyl group, an acylamino group, or a sulfamoyl group,
R² represents a hydrogen atom, a substituted or unsubstituted C₁₋₆ alkyl group, or a halogen atom,
R⁴ and R⁵ are the same as or different from each other, and represent a hydrogen atom, a substituted or unsubstituted C₁₋₆ alkyl group, a substituted or unsubstituted nitrogen-containing heterocyclic ring, a substituted or unsubstituted fused aromatic heterocyclic ring, an acyl group, or an acylamino group; or
R⁴ and R⁵ form a substituted or unsubstituted heterocyclic ring, or a substituted or unsubstituted fused aromatic heterocyclic ring, together with an adjacent nitrogen atom; or
R⁴ and R⁵ represent a substituted or unsubstituted cycloalkylidene amino group, or a substituted or unsubstituted aromatic ring fused cycloalkylidene group.

7. The pharmaceutical composition according to any of claims 1 to 6,
which contains or is used in combination with an agent that reduces production of NADPH in cancer cells.

8. A method for inducing programmed cell death in cancer cells, the method comprising
enhancing production of nitric oxide in cancer cells.

9. The method according to claim 7,
wherein enhancement of production of nitric oxide is enhancement of production of nitric oxide via a transmembrane protein.

10. The method according to claim 7 or 8,
wherein the cancer cells are cancer cells whose ATP production is dependent on glycolysis rather than oxidative phosphorylation.

11. The method according to any of claims 7 to 9,
wherein the cancer cells are cancer cells in which a Warburg effect is expressed.

12. The method according to any of claims 7 to 10,
wherein production of nitric oxide is enhanced by causing the pharmaceutical composition according to any of claims 1 to 6 to come into contact with cancer cells, or administering, to a living body having cancer cells, the pharmaceutical composition according to any of claims 1 to 6.

13. The method according to any of claims 8 to 12, further comprising
introducing, into cancer cells, an agent that reduces production of NADPH in cancer cells.

14. The method according to claim 12,
wherein enhancement of production of nitric oxide further includes causing a substrate of a nitric oxide synthase and/or a substance that serves as a substrate of a nitric oxide synthase to come into contact with cancer cells, or administering, to a living body having cancer cells, a substrate of a nitric oxide synthase and/or a substance that serves as a substrate of a nitric oxide synthase.

15. Use of a compound that enhances production of nitric oxide in cancer cells, in a method for preventing, improving, suppressing progression of, and/or treating cancers.

16. The use according to claim 14,
wherein enhancement of production of nitric oxide is enhancement of production of nitric oxide via a transmembrane protein.

17. The use according to claim 14 or 15,
wherein the cancer cells are cancer cells whose ATP production is dependent on glycolysis rather than oxidative phosphorylation.

18. The use according to any of claims 14 to 16,
wherein the cancer cells are cancer cells in which a Warburg effect is expressed.

19. The use according to any of claims 14 to 17, further comprising use of an agent that reduces production of NADPH in cancer cells.

20. Use of the pharmaceutical composition according to any of claims 1 to 6,
in prevention, amelioration, suppression of progression of, and/or treatment of cancers.

21. The use according to claim 20, further comprising
use of an agent that reduces production of NADPH in cancer cells.

22. A method for preventing, improving, suppressing progression of, and/or treating cancers, the method comprising
administering, to a subject, the pharmaceutical composition according to any of claims 1 to 6.

23. The method according to claim 22, further comprising
administering, to a subject, an agent that reduces production of NADPH in cancer cells.

24. The method according to claim 22, further comprising
administering, to a subject, a substrate of a nitric oxide synthase and/or a substance that serves as a substrate of a nitric oxide synthase.

25. A pharmaceutical composition for preventing, improving, suppressing progression of, and/or treating diseases attributable to a decrease in production of nitric oxide in cells, the pharmaceutical composition containing, as an active component,
a compound represented by Formula (I) defined in claim 5, a compound represented by Formula (III) defined in claim 6, a prodrug thereof, or a pharmaceutically acceptable salt thereof.

26. A pharmaceutical composition for preventing, improving, suppressing progression of, and/or treating a lifestyle disease and/or metabolic syndrome, the pharmaceutical composition containing, as an active component,
a compound represented by Formula (I) defined in claim 5, a compound represented by Formula (III) defined in claim 6, a prodrug thereof, or a pharmaceutically acceptable salt thereof.
